# EUROPEAN PATENT APPLICATION

(11) **EP 4 212 227 A1**
(43) Date of publication of application: **19.07.2023**
(21) Application number: 22152094.3
(22) Date of filing: 18.01.2022
(51) Int. Cl.: B01D 5/00, B01D 19/00, C07C 273/04, C07C 273/16

(54) **LIQUID/VAPOR SEPARATOR**

(71) Applicant: Yara International ASA, 0277 Oslo (NO)
(72) Inventor: Franceus, Danny, 9180 Moerbeke-Waas (BE); Porro, Lino Giovanni, 1040 Etterbeek (BE)
(74) Representative: De Clercq & Partners

(57) **Abstract**

The present disclosure provides a vertically positioned device for separating vapor from a liquid comprising: a container for holding a first liquid/vapor composition; a container inlet for a first liquid/vapor composition; a first container outlet for a gas composition; a conduit for a second liquid/vapor composition comprising, at one end, a conduit inlet and, at the other end, a conduit outlet, wherein the conduit is passing through the container; and a second container outlet for a vapor-depleted liquid composition. The present disclosure also provides an arrangement comprising a carbamate decomposer, a vertically positioned device according to the present disclosure, a pre-condenser, and a condenser; a method for modifying a ureaproducing plant comprising a liquid/vapor separator fluidly connected to a carbamate decomposer, a condenser, and a pipe for carrying a vapor-depleted liquid composition; and a method for revamping a liquid/vapor separator comprising an outlet for a gas composition located in the lower half of the separator.

## Description

### Field of the invention

The present invention is related to the field of chemical engineering, in particular to the field of liquid/vapor separators.

### Background of the invention

A liquid/gas separator, or liquid/vapor separator is an equipment designed to receive a liquid composition comprising dissolved gas therein or a two-phase composition comprising a liquid component and a vapor or gas component, and to separate the dissolved gas from the liquid composition.

A conventional liquid/vapor separator comprises an inlet for the liquid composition to be processed, an outlet for a liquid composition depleted of gas or vapors, and an outlet for the gas or vapors removed from the liquid composition.

In urea-producing plants, liquid compositions can comprise urea, water, free ammonia (NH₃), and/or ammonium salts, such ammonium carbamate, ammonium carbonate and ammonium bicarbonate. To increase the urea content of the liquid composition, ammonium carbamate can be decomposed back to free ammonia and carbon dioxide (CO₂) which are vaporized, together with some water. Such vapors must be removed from the liquid compositions to be recycled. Once the free ammonia and/or free carbon dioxide and/or water are removed from the liquid compositions, they are often re-condensed as an aqueous solution. This condensation is exothermic, i.e. generating heat, and it might be interesting to recover this heat to save energy consumption in the plant. This may be done by connecting the vapor outlet of the liquid/vapor separator to a condenser, or a pre-condenser, comprising a heat recovery system. The heat recovery system may be a device that generates steam from the heat produced by the condensation or a device that is concentrating an aqueous solution, for example an aqueous urea solution, by removing water from the aqueous solution.

Industrial plants, such as urea-producing plants, comprise many equipment and are designed to use as little space as possible to reduce costs and environmental impact, so when one wants to modify an existing plant, for example install a new condenser, there might be severe physical constraints, i.e. lack of space.

There is a need to design a compact liquid/vapor separator that can be connected to a condenser or series of condensers.

### Summary of the invention

The present disclosure provides a device that separate vapors or gases from a liquid composition and that can be connected to an additional condenser.

In a first aspect, the present disclosure provides a vertically positioned device for separating vapor from a liquid, comprising: a container for holding a first liquid/vapor composition; a container inlet for a first liquid/vapor composition; a first container outlet for a gas composition; a conduit for a second liquid/vapor composition comprising, at one end, a conduit inlet and, at the other end, a conduit outlet, wherein the conduit is passing through the container; and a second container outlet for a vapor-depleted liquid composition.

In another aspect, the present disclosure also provides an arrangement comprising a carbamate decomposer, a vertically positioned device according to the present disclosure, a pre-condenser, and a condenser.

In another aspect, the present disclosure provides a method for modifying a urea-producing plant comprising a liquid/vapor separator fluidly connected to a carbamate decomposer, a condenser, and a pipe for carrying a vapor-depleted liquid composition, comprising the steps of: a) disconnecting the liquid/vapor separator from the carbamate decomposer, the condenser and the pipe for carrying away a vapor-depleted liquid composition; b) installing a vertically positioned device according to the present disclosure; c) installing a pre-condenser comprising an inlet and an outlet; and d) connecting: (i) the carbamate decomposer with the container inlet of the device; (ii) the inlet of the pre-condenser with the first container outlet of the device; (iii) the outlet of the pre-condenser with the conduit inlet of the device; and (iv) the conduit outlet of the device with the inlet of the condenser.

### Brief description of the figures

The following description of the figure of a specific embodiment of a system according to the present disclosure is only given by way of example and is not intended to limit the present explanation, its application or use. In the figure, identical reference numerals refer to the same or similar parts and features.
Figure 1 describes a conventional liquid/vapor separator
Figure 2 describes an embodiment of a device according to the present disclosure.
Figure 3 describes an arrangement comprising an embodiment of a device according to the present disclosure.

### Detailed description of the invention

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

In a first aspect, the present disclosure provides a vertically positioned device for separating a vapor or a gas from a liquid comprising: a container for holding a first liquid/vapor composition; a container inlet for a first liquid/vapor composition; a first container outlet for a gas composition; a conduit for a second liquid/vapor composition comprising, at one end, a conduit inlet and, at the other end, a conduit outlet, wherein the conduit is passing through the container; and a second container outlet for a vapor-depleted liquid composition.

The device is configured to receive a first liquid/vapor composition, i.e. a composition comprising a liquid component and a vapor component. This composition comprises volatile components, which may be dissolved in the liquid or be present as vapors in the composition. In a urea-producing plant, the vapors comprised in the composition directed to the device include ammonia, carbon dioxide, water and inert gasses or inerts. Inerts may comprise hydrogen gas, methane, nitrogen and oxygen.

The device comprises a container that can hold a first liquid/vapor composition in operation. The container may be made in a material suitable for the operating conditions. The choice of material may be dictated by the nature of the composition to contain, for example if the composition contains corrosive chemicals. In one embodiment, the container is made of a metal, in particular stainless steel.

The device comprises a container inlet for a first liquid/vapor composition. In a production system, the container inlet is connected to a device that produces a first liquid/vapor composition, such as a carbamate decomposer in a urea-producing plant. The inlet is located on the container wall.

In one embodiment, the inlet is located above the liquid level in operation. This facilitates the flow of the first liquid/vapor composition into the container. The liquid level in operation is determined by operating conditions, and, in particular, determines the residence time of the liquid in the container. The residence time needs to be long enough so that the free gas and vapors present in the liquid has enough time to escape the liquid. The separation rate of the vapors from the liquid depends on operating conditions, such as pressure and temperature in the device, the amount of vapors comprised in the first liquid/vapor composition, the size and geometry of the container.

The device comprises two outlets: a first container outlet for a gas composition, and a second container outlet for a vapor-depleted liquid composition. The first container outlet for a gas composition is arranged to be connected to a first condenser, also named pre-condenser, in particular to an inlet of a pre-condenser. In the pre-condenser, the gas composition that leaves the device is cooled down and partially condensed, forming a second liquid/vapor composition, and heat from condensation is recovered, for example in the form of steam or by evaporating water from an aqueous solution, in particular from an aqueous urea solution. In a urea-producing plant, the composition leaving the device via the first container outlet for a gas composition may comprise any one of ammonia, carbon dioxide, water and inerts.

The device comprises a second container outlet for a vapor-depleted liquid composition. The second container outlet is used to recover the liquid composition, which is depleted of gas/vapor(s). The second container outlet may be located at the bottom of the container, so that the liquid composition may flow out of the container without the need for mechanical assistance such as a pump.

The device also comprises a conduit for a second liquid/vapor composition comprising, at one end, a conduit inlet and, at the other end, a conduit outlet, wherein the conduit is passing through the container. In a production system, the conduit for a second liquid/vapor composition of the device is arranged to be connected, at the conduit inlet, to the outlet of the pre-condenser to receive the partially condensed composition, wherein the inlet of that pre-condenser is arranged to be connected to the first container outlet for a gas composition of the device. The conduit is positioned inside the container, but it does not exchange matter with the container.

In a production system, the conduit outlet is connected to a second condenser, which cools down and condenses further the second liquid/vapor composition produced by the pre-condenser.

In one embodiment, the conduit outlet is located at the bottom of the container or, in operation, below the liquid level. The position of the conduit outlet may be imposed by the layout of the plant. A conduit outlet located at the bottom of the container or, in operation, below the liquid level, may have the advantage that the second condenser, which may be a vertically positioned device, can be located directly under the liquid/vapor separator, thus minimizing the space occupied by the production assembly comprising the separator and the condenser.

In another aspect, the present disclosure also provides a urea-producing plant comprising the device according to the present disclosure.

A urea-producing plant uses ammonia and carbon dioxide as raw materials and produce urea in liquid or solid form. There are different designs of urea-producing plants, but they all operate according to some common principles: firstly, ammonia and carbon dioxide are reacted in a high-pressure section that generates an aqueous composition comprising urea, ammonium salts, such as ammonium carbonate, ammonium bicarbonate, and/or ammonium carbamate, and optionally free ammonia. This aqueous composition undergoes one or more purification and concentration steps, and a concentrated urea solution, sometimes called urea melt, is obtained. The concentrated urea solution can be transformed in a final product, which may be liquid or solid.

In one embodiment, the urea-producing plant comprises a high pressure section and any one of a medium-pressure section, a low-pressure section, a concentration section, a finishing section, or a granulation section.

An important equipment found in urea-producing plant is a carbamate decomposer, wherein an aqueous solution comprising urea and ammonium carbamate is heated up, so that ammonium carbamate decomposes back into ammonia and carbon dioxide. Since ammonia and carbon dioxide are gases in a broad range of temperature and pressure, it is possible to separate these from an aqueous solution. A urea-producing plant may comprise several carbamate decomposers. For example a plant comprising a high pressure section and a low pressure section may comprise a carbamate decomposer in the high pressure section, and a carbamate decomposer in the low pressure section.

The high-pressure section is the first section of a urea plant located directly downstream after the urea reactor. The pressure in the high-pressure section is typically above 100 bar.

The medium-pressure section refers to the section of a urea plant wherein the pressure of the fluids is comprised between 30 and 70 bar.

The low-pressure section refers to the section of a urea plant wherein the pressure of the fluids is below 10 bar.

The separation of vapors from a liquid is performed in a liquid/vapor separator. The working principle is quite simple: the solution comprising urea and vapors is brought into a container where the solution is allowed to stay for from a few second to up to a few minutes, in particular from 10 to 60 seconds, and separation is obtained by gravity and/or by centrifugal separation. The operating conditions of the separator, in particular the pressure and temperature inside the separator, the fluid-dynamics and geometry of the separators are such that vapors are separated from the solution, leaving an aqueous solution of urea depleted of vapor(s).

The gas composition exiting the device via the first container outlet comprise ammonia, carbon dioxide, water and optionally inerts. This gas composition needs then to be condensed back to an aqueous solution, and this is done by directing the gaseous composition to a condenser, where a cooling liquid cools down the gases, causing the water vapors to condense to liquid water, and ammonia and carbon dioxide dissolve then into the liquid, and react to form ammonium ions. However, in such a system, the heat given off by the gaseous composition to the cooling water cannot be used and is considered lost. In such a system, the condenser can be placed directly under the separator to save space. A prior art separator comprises an inner tube, in particular a straight inner tube wherein the top end of the tube is located above the liquid level in operation, and the bottom end of the tube is a gas outlet located at the bottom of its container or at least below the liquid level during operation. During condensation of the gas composition in the condenser, an under pressure, or vacuum, is created in the condenser and draws the gas composition from the separator to the condenser.

In one aspect, the present disclosure provides an arrangement comprising a carbamate decomposer comprising an outlet for a first liquid/vapor composition, a vertically positioned device according to the present disclosure, a pre-condenser comprising an inlet for a gas composition and an outlet for a second liquid/vapor composition, and a condenser comprising an inlet for a second liquid/vapor composition, wherein:
- the outlet of the carbamate decomposer is fluidly connected to the container inlet of the device;
- the first container outlet of the device is connected to the inlet of the pre-condenser;
- the outlet of the pre-condenser is fluidly connected to the conduit inlet of the device; and
- the conduit outlet of the device is fluidly connected to the inlet of the condenser.

It is possible to recover some energy contained in the gaseous composition produced by the separator using a pre-condenser, wherein water absorbs heat from the gaseous composition, and for example evaporating the water to generate steam at low pressure, for example from 1.5 to 5.0 bars. This is called a double effect. The steam produced in the first condenser or pre-condenser can be used in the urea-producing plant, such a plant comprises a lot of equipment that require steam to operate. A urea-producing plant requires steam to operate. Steam is often produced by heating water in boilers, in particular in boilers using non-renewable energy sources, such as oil or natural gas, that produce carbon dioxide. The generation of steam in the pre-condenser allows the plant to reduce the production of steam from boilers, therefore save costs and reduce greenhouse gas emissions.

In some cases, the temperature of the gas composition leaving the liquid/vapor separator may not be high enough to produce steam under the required pressure. In such cases, the heat given off by the condensation can be used to directly evaporate water contained in an aqueous solution, in particular an aqueous urea solution, without using steam as an intermediate heat transfer agent.

The installation of a pre-condenser also requires a new liquid/vapor separator with at least one additional inlet and one additional outlet. Industrial plants, such as urea-producing plants, are usually built as compact as possible to limit the footprint or the plant and reduce the need for pipes transporting the different compositions. So it may be challenging to install two new pieces of equipment due to lack of space. It was found that it was possible to modify an existing liquid/vapor separator to accommodate the new pre-condenser.

A new gas outlet to be connected to the pre-condenser is created or an existing outlet may also be re-purposed. This outlet may be located above the liquid level during operation. The top end of the inner tube connected to the gas outlet connected to the condenser may be modified: a new inlet for a second liquid/vapor composition is created on the side or the top of the container and is connected to the existing inner tube to connect the new inlet, thereby creating a conduit having a conduit inlet, the new inlet for a second liquid/vapor composition, and a conduit outlet, being the inlet for a gas of the original liquid/vapor separator, and the conduit outlet is connected to the condenser. The new conduit inlet of the separator is then fluidly connected to the outlet of the pre-condenser.

In the assembly according to the present disclosure, the composition comprising a liquid and free gases as vapors or dissolved in the liquid, enters the separator as in the conventional design of the prior art. The gases/vapors separate from the solution and are drawn to the new container outlet of the container that is connected to the pre-condenser. In the pre-condenser, the temperature of the gases is lowered, some water vapor is condensed into liquid water, some ammonia and carbon dioxide are condensed as carbamate solution, and heat is released. The cooled, second liquid/vapor composition produced by the pre-condenser enters the conduit inlet of the separator and is directed to the condenser without mixing with the composition present in the container.

Cooling the gaseous composition in the pre-condenser creates an under pressure and draws in the vapors from the separator to the pre-condenser.

In another aspect, the present disclosure provides a method for modifying a urea-producing plant comprising a liquid/vapor separator fluidly connected to a carbamate decomposer, a condenser, and a pipe for carrying a vapor-depleted liquid composition, comprising the steps of: a) disconnecting the liquid/vapor separator from the carbamate decomposer, the condenser and the pipe for carrying away a vapor-depleted liquid composition; b) installing a vertically positioned device according to the present disclosure; c) installing a pre-condenser comprising an inlet and an outlet; and d) connecting: (i) the carbamate decomposer with the container inlet of the device; (ii) the inlet of the pre-condenser with the first container outlet of the device; (iii) the outlet of the pre-condenser with the conduit inlet of the device; and (iv) the conduit outlet of the device with the inlet of the condenser.

The method allows the plant to reduce its consumption of steam by recovering energy from the condensation of the vapors, and this reduces the energy required by the plant to operate and save costs and reduce greenhouse gas emissions in plants where steam is generated by burning fossil fuels. The revamping method first requires to disconnect the existing liquid/vapor separator from the assembly, notably the condenser, the carbamate decompose and any pipe connected to it, such as the pipe connected to the liquid outlet of the separator. A device according to the present disclosure is then installed at the location of the previously installed separator. In most cases, the liquid/vapor separator present in the plant can be modified to obtain one according to the present disclosure. A new gas outlet can be created and the inner tube can be modified to be connected to a new inlet that is arranged to be connected to the new pre-condenser. Next, a pre-condenser comprising an inlet and an outlet is installed where practically possible; and finally several connections are performed: (i) the carbamate decomposer with the container inlet of the device; (ii) the inlet of the pre-condenser with the first container outlet of the device; (iii) the outlet of the pre-condenser with the conduit inlet of the device; and (iv) the conduit outlet of the device with the inlet of the condenser.

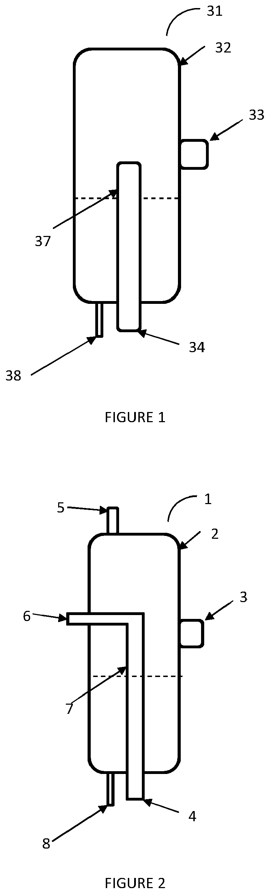

Figure 1 shows a conventional liquid/vapor separator. The device 31 comprises a container 32 for holding a first liquid/vapor composition, a first inlet 33 for a first liquid/vapor composition, a first outlet 34 for a vapor composition, a second outlet 38 for a liquid composition depleted of vapors, and an inner tube 37 connected to the first outlet 34. The hash line shows the level of the liquid in the liquid/vapor separator in operation. The inner tube 37 has an open top end, such that vapors can exit the container 32 via the tube 37 and the outlet 34. The outlet **34** is connected to a condenser where the vapors are cooled down and at least partially condensed.

Such conventional liquid/vapor separator can be easily converted to a device according to the present invention. A new outlet for a gas composition can be installed at the top of the container, and this new outlet can be connected to a pre-condenser. A new inlet for a second liquid/vapor composition can be installed on the side of the container, in particular at the same height as the top end of the inner tube **37**. The inner tube **37** can be transformed into a conduit 7 connecting the new inlet for a second liquid/vapor composition, and the outlet 4.

In another aspect, the present disclosure provides a method for revamping a liquid/vapor separator comprising an outlet for a gas composition located in the lower half of the separator, the method comprising the steps of: a) installing a new outlet for a gas composition on the container of the separator; b) installing a new conduit inlet for a second liquid/vapor composition on the container of the separator;
c) extending the pipe to the new conduit inlet, thereby forming a conduit between the new conduit inlet and the existing outlet of the separator.

The pre-condenser can be installed in any location in the plant, preferably not too far from the existing carbamate condenser, separator and condenser, and is connected to the device according to the present disclosure with pipes.

In another aspect, the present disclosure provides a method for removing vapors from a first liquid/vapor composition with a device according to the present invention comprising the steps of:
a) providing a first liquid/vapor composition to the container inlet of the device;
b) directing the gas composition from the first container outlet of the device to the inlet of a pre-condenser;
c) partially condensing the gas composition in the pre-condenser; thereby forming a second liquid/vapor composition; and
d) directing the second liquid/vapor composition produced by the pre-condenser to the conduit inlet of the device, the conduit inlet of the device being fluidly connected to a condenser via the conduit and the conduit outlet of the device.

The method is practiced in the system described above.

A first liquid/vapor composition is provided to the container inlet of the liquid/vapor separator, and enters the container. In the container, the vapors separate from the liquid phase. The vapors present in the container are drawn to the pre-condenser since the pressure in the pre-condenser is lower than the pressure in the container due to the cooling effect of the pre-condenser.

In one embodiment, the pre-condenser is a top-to-bottom device: the pre-condenser is a vertically positioned device, and the hot vapors are entering the pre-condenser at the top of the pre-condenser and travel downwards.

In the pre-condenser, the temperature of the vapors is decreased causing some of the vapors, in particular the water vapors and ammonia carbon dioxide vapors, to condensate into a liquid phase. Not all the vapors condensate, so the composition obtained at the other end, in particular the bottom end, of the pre-condenser is a two-phase liquid/vapor composition, also named second liquid/vapor composition.

The second liquid/vapor composition travels from the pre-condenser to the conduit of the liquid/vapor separator to the condenser connected to the conduit, where it undergoes a second and final cooling and condensing step.

Figure 2 shows an embodiment of a device according to the present disclosure. The device 1 comprises a container 2 for a first liquid/vapor composition, a container inlet 3 for a first liquid/vapor composition, a first container outlet 5 for a gas composition, a conduit 7 for a second liquid/vapor composition comprising, at one end, a conduit inlet 6 and, at the other end, a conduit outlet 4, wherein the conduit 7 is passing through the container 2, and a second container outlet 8 for a vapor-depleted liquid composition. A conventional device according to figure 1 was installed in a urea-producing plant located in Ferrara in Italy and operated by Yara with a production capacity of 1750 tons of urea per day. The conventional device was replaced with a device according to figure 2. The new device was connected to a new pre-condenser, which used the heat released by the condensation to evaporate water from a urea aqueous solution. The heat released by the condensation was found equivalent to 3.7 Gcal per hour of 4 bar steam. The hash line shows the level of the liquid in the liquid/vapor separator in operation.

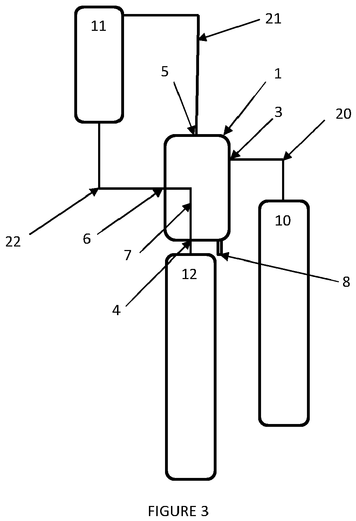

Figure 3 shows a part of a urea plant comprising a liquid/vapor separator according to the present disclosure. The urea plant comprises a carbamate decomposer 10, a pre-condenser 11, a condenser 12, and a liquid/vapor separator 1 according to the present disclosure. The outlet of the carbamate decomposer 10 is connected via the pipe 20 to the first inlet 3 for a first liquid/vapor composition of the device 1. The second outlet 5 for a gas composition of the device 1 is connected via the pipe 21 to the inlet of the pre-condenser 11. The outlet of the pre-condenser 11 is connected to the inlet of the condenser 22, wherein the connection comprises the second inlet 6 of the device 1, the inner tube 7 of the device 1 and the first outlet 4 of the device 1.

## Claims

1. A vertically positioned device (1) for separating vapor from a liquid comprising:
- a container (2) for holding a first liquid/vapor composition;
- a container inlet (3) for a first liquid/vapor composition;
- a first container outlet (5) for a gas composition;
- a conduit (7) for a second liquid/vapor composition comprising, at one end, a conduit inlet (6) and, at the other end, a conduit outlet (4), wherein the conduit (7) is passing through the container (2); and
- a second container outlet (8) for a vapor-depleted liquid composition.

2. The device (1) according to claim 1, wherein the conduit outlet (4) is located at the bottom of the container (2) or, in operation, below the liquid level.

3. The device (1) according to claim 1 or 2, wherein the first container outlet (5) is located at the top of the container (2) or above the liquid level in operation.

4. The device (1) according to any one of claims 1 to 3, wherein the second container outlet (8) is located at the bottom of the container (2).

5. An arrangement comprising a carbamate decomposer (10) comprising an outlet for a first liquid/vapor composition, a vertically positioned device (1) according to any one of claims 1 to 4, a pre-condenser (11) comprising an inlet for a gas composition and an outlet for a second liquid/vapor composition, and a condenser (12) comprising an inlet for the second liquid/vapor composition, wherein:
- the outlet of the carbamate decomposer is fluidly connected to the container inlet (3) of the device (1);
- the first container outlet (5) of the device (1) is fluidly connected to the inlet of the pre-condenser (11);
- the outlet of the pre-condenser (1) is fluidly connected to the conduit inlet (6) of the device (1); and
- the conduit outlet (4) of the device (1) is fluidly connected to the inlet of the condenser (12).

6. The arrangement according to claim 5, wherein the vertically positioned device (1) is located directly above the condenser (12).

7. A method for modifying a urea-producing plant comprising a liquid/vapor separator fluidly connected to a carbamate decomposer (10), a condenser (12), and a pipe for carrying a vapor-depleted liquid composition, comprising the steps of:
a) disconnecting the liquid/vapor separator from the carbamate decomposer (10), the condenser (12) and the pipe for carrying away a vapor-depleted liquid composition;
b) replacing the liquid/vapor separator with a vertically positioned device (1) according to any one of claims 1 to 4; or revamping the liquid/vapor separator into a device (1) according to any one of claims 1 to 4;
c) installing a pre-condenser (11) comprising an inlet and an outlet; and
d) connecting:
(i) the carbamate decomposer with the container inlet (3) of the device (1); (ii) the inlet of the pre-condenser (11) with the first container outlet (5) of the device (1);
(iii) the outlet of the pre-condenser (11) with the conduit inlet (6) of the device (1); and
(iv) the conduit outlet (4) of the device (1) with the inlet of the condenser (12).

8. A method for removing vapors from a first liquid/vapor composition with a device (1) according to any one of claims 1 to 4 comprising the steps of:
a) providing a first liquid/vapor composition to the container inlet (3) of the device (1);
b) directing the gas composition from the first container outlet (5) of the device (1) to the inlet of a pre-condenser (11);
c) partially condensing the gas composition in the pre-condenser (11); thereby forming a second liquid/vapor composition; and
d) directing the second liquid/vapor composition produced by the pre-condenser (11) to the conduit inlet (6) of the device (1), the conduit inlet (6) of the device (1) being fluidly connected to a condenser (12) via the conduit (7) and the conduit outlet (4) of the device (1).

9. A method for revamping a liquid/vapor separator (31) comprising an outlet (34) for a gas composition located in the lower half of the separator (31), the method comprising the steps of:
a) installing a new outlet (5) for a gas composition on the container (32) of the separator (31);
b) installing a new conduit inlet (6) for a second liquid/vapor composition on the container (32) of the separator (31);
c) extending the pipe (7) to the new conduit inlet (6), thereby forming a conduit between the new conduit inlet (6) and the outlet (34).
